# EUROPÄISCHE PATENTSCHRIFT

(11) **EP 1 369 090 B1**
(45) Veröffentlichungstag und Bekanntmachung des Hinweises auf die Patenterteilung: **15.12.2010**
(21) Anmeldenummer: 02011600.0
(22) Anmeldetag: 28.05.2002
(51) Int. Cl.: A61B 19/00

(54) **Navigations-Kalibrierung medizinischer Instrumente bzw. Implantate**
Calibration of a navigation system for medical instruments and implants
Etalonnage d'un système de navigation pour instruments et d'implants chirurgicaux

(43) Veröffentlichungstag der Anmeldung: 10.12.2003
(73) Patentinhaber: BrainLAB AG, 85622 Feldkirchen (DE)
(72) Erfinder: Zeiss, Mario, 85586 Poing (DE); Blau, Arno, 85622 Feldkirchen (DE); Birkenbach, Rainer, 85586 Poing (DE); Seifferth, Falko, 85604 Zorneding (DE)
(74) Vertreter: Schwabe - Sandmair - Marx

(56) Entgegenhaltungen:
- WO-A-01/67979
- US-A- 5 921 992
- US-A- 6 112 113
- US-A1- 2001 034 530

## Beschreibung

Die vorliegende Erfindung betrifft die Navigations-Kalibrierung nicht rotationssymmetrischer, medizinischer Instrumente.

Die moderne, computergestützte Chirurgie ist in der Lage, während der Operation Instrumente oder Implantate des Chirurgen auf einem Monitor in Relation zu anatomischen Daten darzustellen, die vorher aus einem Patientenscan (CT, MR) erhalten wurden. Dazu müssen Informationen über das Instrument, beispielsweise über dessen räumliche Position und über die Lage des funktionellen Abschnittes, beispielsweise der Spitze des Instruments in dem verwendeten medizinischen Navigationssystem bekannt sein, d. h. die Instrumente bzw. die Implantate müssen kalibriert werden. Hierbei gibt es die Möglichkeit, eine Vorkalibrierung (prä-operative Kalibrierung) durchzuführen, d. h. dem Navigationssystem schon vorab die geometrischen Daten des Instruments bzw. des Implantats bekannt zu machen und softwareseitig fest zu speichern. Eine weitere Möglichkeit ist die sogenannte intra-operative Kalibrierung, bei der das Instrument bzw. das Implantat während der Operation vom behandelnden Personal kalibriert wird.

Die Verwendung von prä-operativ kalibrierten Instrumenten bzw. Implantaten macht es in vielen Fällen trotzdem notwendig, intra-operativ die Genauigkeit der Instrumente zu überprüfen und eine Neukalibrierung durchzuführen. Ein weiterer Vorteil der intra-operativen Kalibrierung liegt darin, dass auf vorhandene Instrumente zurückgegriffen werden kann und man nicht auf die Instrumente angewiesen ist, deren Geometrie softwareseitig schon gespeichert ist. Mit anderen Worten kann jeder Chirurg beispielsweise seine eigenen Instrumente verwenden. Eine intra-operative Kalibrierung ist auch dann sehr wichtig, wenn sich Instrumente zwischen zwei Operationen verändert haben (zum Beispiel durch das Nachschleifen eines Instruments), jedoch während der Operation hochgenau kalibriert sein müssen.

Aus der US 6,112,113, der US 2001/0034530 A1, der WO 01/67979 und der US 5,921,992 sind Positionsbestimmungen für Instrumente mit Spitzen bekannt.

Eine intra-operative Kalibrierung kann auf verschiedene Arten erfolgen. Bei einer Kalibrierungsart nach dem Stand der Technik wird nur eine sogenannte Punktkalibrierung durchgeführt, d. h. es wird nur die Länge des Instruments bzw. Implantats bestimmt, aber nicht dessen Geometrie. Eine zweite und weiterführende Methode besteht darin, neben der Länge auch den exakten Vektor des Instruments, d. h. seine Geometrie zu bestimmen. Hierbei werden unterschiedliche Verfahren und Hilfsmittel eingesetzt, die sich jedoch derzeit sämtlich auf rotationssymmetrische Instrumente beschränken, welche sich sehr einfach kalibrieren lassen. Zu solchen Techniken wird auf die US 6,021,343, die WO 96/11624 und die US 5,921,922 hingewiesen. Aus letzterer Schrift ist eine intra-operative Kalibrierung von Instrumenten bekannt. Die Instrumente werden zur Kalibrierung während der Operation oder kurz vor der Verwendung in ein festes Kalibrierungstool eingespannt, also in eine Patienten- bzw. ortsfeste Einrichtung, in die das Instrument eingebracht wird, um es zu kalibrieren.

Der Nachteil des in der US 5,921,992 beschriebenen Systems liegt wiederum darin, dass eine Kalibrierung lediglich in Bezug auf die Ausrichtung des Instrumentes und dessen punktförmiger Spitze vorgenommen wird. Die im Rahmen dieser Offenbarung für die Kalibrierung geeigneten Instrumente sind lediglich solche, bei denen die Position der Instrumentenspitze sowie des anschließenden linear verlaufenden Abschnittes von Wichtigkeit bei der Behandlung sind.

Es ist die Aufgabe der vorliegenden Erfindung, eine Kalibrierung auch für solche Instrumente zu ermöglichen, die nicht im Wesentlichen rotationssymmetrisch sind bzw. eine im Wesentlichen punktförmige Spitze haben. Insbesondere sollen komplex gestaltete Instrumente und Implantate für den optimalen Einsatz im Rahmen medizinischer Behandlungen kalibriert werden können.

Diese Aufgabe wird erfindungsgemäß durch ein Verfahren gemäß dem beiliegenden Anspruch 1 und eine Vorrichtung gemäß dem beiliegenden Anspruch 8 gelöst. Die Unteransprüche definieren bevorzugte Ausführungsformen der Erfindung.

Das Verfahren gemäß der vorliegenden Erfindung zur Kalibrierung bzw. Registrierung medizinischer Instrumente ist seiner Gattung nach eines, bei dem eine räumliche Position des Instruments mittels eines medizinischen Navigationssystems ermittelt wird, um die Relativposition des Instruments gegenüber anatomischen Daten zu ermitteln. Da bei kompliziert ausgestalteten Instrumenten eine Information über eine Spitzenposition bzw. eine einzige dargestellte Raumrichtung nicht ausreicht, um das Instrument optimal in die Navigation einzubinden, wird gemäß der vorliegenden Erfindung die räumliche Ausrichtung eines linien-oder ebenenförmigen, funktionellen Abschnitts des Instruments ermittelt. Mit anderen Worten wird eine mehrdimensionale Kalibrierung des Instruments vorgenommen, es wird beispielsweise die Lage einer Ebene bzw. einer Kante des Instruments bestimmt, wenn diese von einer funktionellen Art ist, also falls es für die Funktion des Instrumentes darauf ankommt, wie diese Ebene oder Kante im Raum angeordnet ist. Man könnte die erfindungsgemäße Kalibrierung auch als eine Kalibrierung von nicht rotationssymmetrischen Instrumenten bezeichnen, und der besondere Vorteil der Erfindung beruht darin, dass eine optimierte Navigation solcher Instrumente ermöglicht wird, was auch das Endresultat, beispielsweise die korrekte Schaffung eines Flächenschnittes mit einem Instrument verbessert. Mit der vorliegenden Erfindung wird es somit möglich, auch komplizierter gestaltete Instrumente intra-operativ zu kalibrieren und damit die computergestützte Navigation auch hierfür nutzbar zu machen.

Gemäß der vorliegenden Erfindung wird ein geeigneter Abschnitt des Instruments an einem Kalibrierungsabschnitt eines Kalibrierungshilfsmittels ausgerichtet, dessen räumliche Ausrichtung im Navigationssystem bekannt ist und der getrackt wird.

Wenn als Instrument beispielsweise ein chirurgischer Meißel zu verwenden ist, ist es vorteilhaft, die räumliche Ausrichtung der Schneidenfläche dieses Meißels zu ermitteln.

Es kann insbesondere der funktionelle Abschnitt selbst sein, der an dem Kalibrierungshilfsmittel bzw. an dessen Kalibrierungsabschnitt ausgerichtet wird, wobei dieser Kalibrierungsabschnitt bei einer Ausführungsform eine Linienform aufweisen kann, insbesondere eine Liniennut. Die Schneidenfläche eines chirurgischen Meißels kann beispielsweise in einer solchen Liniennut ausgerichtet werden.

Der oben angesprochene Kalibrierungsabschnitt kann im Rahmen der vorliegenden Erfindung aber auch eine Ebene am Kalibrierungshilfsmittel sein.

In weiterer Ausgestaltung des erfindungsgemäßen Verfahrens wird die räumliche Ausrichtung des mehrdimensional ausgestalteten, funktionellen Abschnittes des Instruments mit Hilfe schon vorhandener Informationen über die Ausrichtung einer bestimmten anderen, charakteristischen Linie oder Ebene für das Instrument ermittelt.

Im Rahmen der vorliegenden Erfindung besteht ferner die Möglichkeit, die räumliche Ausrichtung des funktionellen Abschnitts des Instruments mit Hilfe der Registrierung mehrerer Punkte auf dem Instrument durch ein schon kalibriertes Registrierungsinstrument zu ermitteln.

Die erfindungsgemäße Vorrichtung zur Kalibrierung bzw. Registrierung medizinischer Instrumente umfasst ein medizinisches Navigationssystem, und ein Kalibrierungshilfsmittel, wobei das Kalibrierungshilfsmittel einen Kalibrierungsabschnitt aufweist, dessen räumliche Ausrichtung im Navigationssystem bekannt ist und an dem ein linien-oder ebenenförmiger, fünktioneller Abschnitt des Instruments ausgerichtet werden kann.

Bei einer Ausführungsform der erfindungsgemäßen Vorrichtung ist der Kalibrierungsabschnitt ein solcher, der die Form einer Liniennut aufweist, während gemäß einer anderen Ausführungsform ein Kalibrierungsabschnitt bereitgestellt wird, der eine Ebene ist.

Die Erfindung wird im Weiteren anhand bevorzugter Ausführungsbeispiele näher erläutert. Es zeigen:
- Figur 1: die Kalibrierung eines chirurgischen Meißels mittels einer Kalibrierungsnut;
- Figur 2: die Kalibrierung eines chirurgischen Meißels mit Hilfe einer Kalibrierungsebene an einem Kalibrierungshilfsmittel; und
- Figuren 3 bis 5: Darstellungen zur erfindungsgemäßen Kalibrierung, die eine konkrete Ausführungsform erläutern.

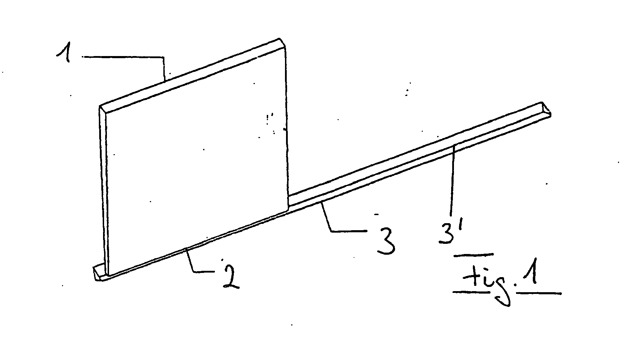

In Figur 1 wird dargestellt, wie die räumliche Ausrichtung der Schneidenfläche eines chirurgischen Meißels erfindungsgemäß kalibriert werden kann. Der Meißel 1 weist eine Schneidenfläche 2 auf. Wenn nun mit diesem Meißel beispielsweise eine Knochenstruktur durchtrennt werden soll, so ist es in manchen Fällen von großer Wichtigkeit, dass die Trennfläche eine bestimmte Ausrichtung hat, insbesondere dann, wenn an dieser Trennfläche spezielle Vorrichtungen angebracht werden sollen, deren Ausrichtung wiederum wichtig ist. Dazu ist es im Rahmen der Navigation notwendig, die Ausrichtung der Schneidenfläche 2 zu kennen, und im Rahmen der Kalibrierung kann eine solche Bestimmung beispielsweise dadurch erfolgen, dass der Meißel 1 mit seiner Schneidenfläche 2 in die Nut 3' des nutförmigen Kalibrierungsinstrumentes 3 eingesetzt wird, wie es in Figur 1 prinzipiell dargestellt ist. Dabei wird die Schneidenfläche 2 des Meißels mit Hilfe der bereits bekannten Geometrie der Nutlinie 3' abgeglichen oder abgetastet. Diese Informationen werden gespeichert und ermöglichen die Darstellung des Meißels während der Behandlung mit Hilfe des Navigationssystems. Die Lage der Nut, die im Computer des Navigationssystems bekannt ist, kann als geometrischer Datensatz auf das zu kalibrierende Instrument übertragen werden.

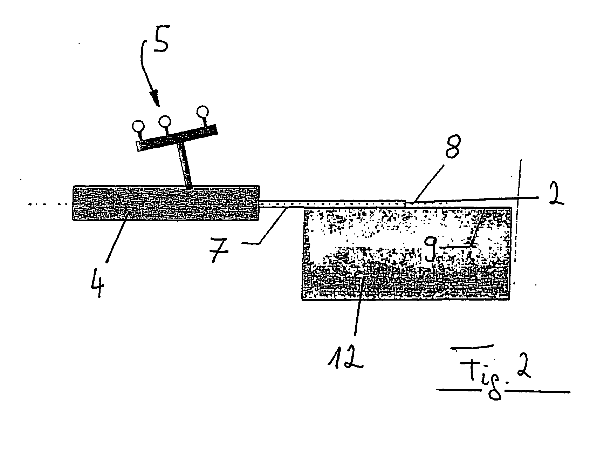

Gemäß der Ausführungsform nach Figur 2 wird ein Kalibrierungstool 12 verwendet, um die Geometrie des Meißels 4 und insbesondere seiner Schneidenfläche 2 im Raum zu bestimmen. Die Referenzierungseinrichtung 5 wird hierbei nicht primär benötigt, sie ist deshalb in schieier Lage dargestellt. Das Kalibrierungshilfsmittel 12 weist eine Ebene 9 auf, deren Lage im Raum bekannt ist. Mit anderen Worten erfolgt hierbei die Bestimmung der Geometrie des Meißels im Raum mit Hilfe einer bekannten Ebene außerhalb des Meißels, wobei nach einer Ausrichtung des Meißels, also beispielsweise einem Auflegen der Schneidenkante 2 auf die bekannte Ebene 9, die Berechnung der Lage der Ebene 8 erfolgen kann, welche die Schneidenfläche 2 enthält, deren Raumrichtung beispielsweise durch ein Verfahren ermittelt wird, wie es anhand der Figur 1 beschrieben wurde.

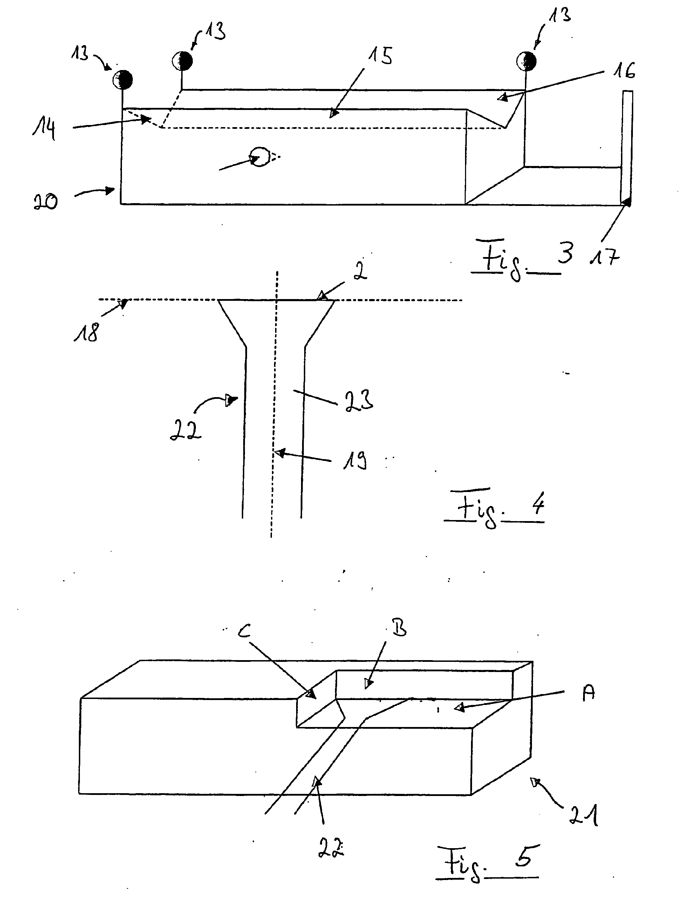

Ein konkretes Ausführungsbeispiel für die erfindungsgemäße Kalibrierung wird nunmehr anhand der Figuren 3 bis 5 erläutert. Die Figur 3 zeigt einen Funktionsteil eines erfindungsgemäßen Kalibrierungsinstruments, der mit dem Bezugszeichen 20 bezeichnet wird. Ein anderer Teil des Kalibrierungsinstruments, der ebenfalls zusammen mit dem Teil 20 bereitgestellt werden kann, ist in Figur 5 gezeigt und mit dem Bezugszeichen 21 versehen.

Der Instrumententeil 20 weist eine V-Nut 14 auf, die von den Nutebenen 15 und 16 begrenzt wird. Ferner ist an der rechten Seite des Instrumententeils 20 noch ein Anschlag 17 vorgesehen.

Mit diesem Kalibrierungsinstrument bzw. mit den Instrumententeilen 20 und 21 (Figuren 3 und 5) soll nunmehr ein Meißel kalibriert werden, dessen Vorderteil in Figur 5 mit dem Bezugszeichen 22 bezeichnet ist. Der Meißel weist die Schneidenkante 2 auf, die auf dem Kantenvektor 18 (Spitzenvektor) liegt sowie einen Griffabschnitt 23 mit dem Achsenvektor 19.

Der Instrumententeil 21 des Kalibrierungsinstruments, der in Figur 5 dargestellt ist, weist als Funktionselemente eine Aussparung auf, die von den Ebenen A, B und C definiert, wobei die Ebene A gegenüber der Oberfläche parallel abgesenkt und die Ebenen B und C senkrecht hierauf und aufeinander senkrecht stehen. Ebenfalls ist der Meißel 22 andeutungsweise dargestellt.

Es soll nun im Weiteren anhand dieses konkreten Ausführungsbeispiels angedeutet werden, in welcher Weise erfindungsgemäß Kalibrierungen für einen solchen Meißel vorgenommen werden können.

Was die Kalibrierung der räumlichen Ausrichtung der Schneidenfläche 2 des Meißels angeht, so kann auch mit dem Kalibrierungsinstrument 20, 21 nach dem anhand von Figur 1 beschrieben Prinzip vorgegangen werden. Dies bedeutet, die Schneidenkante 2 des Meißels wird in ein Nut eingelegt, deren Raumrichtung bekannt ist, beispielsweise in die Nut 14 des Instrumententeils 20 oder in die Schnittlinie der Ebenen A und B des Instrumententeils 21, das ebenfalls lagebekannt ist (beispielsweise über nicht dargestellte Reflektorenanordnungen). Dabei wird die Schneidenkante 2 des Meißels genau in die Nut 14 bzw. an die Kante zwischen der Ebene A und der Ebene B gelegt, und Geometriedaten des Kalibrierungselements werden für die Kalibrierung verwendet. Eine korrekte Positionierung des Meißels relativ zum Kalibrierungsinstrument 20, 21 wird dabei vorausgesetzt.

Die Kalibrierung der Raumrichtung der Schneidenkante kann aber auch lediglich an einer Ebene stattfinden, wenn die Ausrichtung dieser Ebene bekannt ist, wie beispielsweise diejenige der Ebene B in Figur 5. Dabei wird der Meißel, der an seinem Griffabschnitt eine getrackte Referenzierungseinrichtung (Reflektorenanordnung) aufweist, an irgendeiner Stelle an die Ebene B mit der Schneidenkante 2 aufliegend angelegt und eine Rotationsbewegung wird um die Schneidenkante 2 herum durchgeführt. Durch die Rotationsbewegung kann die Rotationsachse (Schneidenkante) im Navigationssystem als Drehachse bestimmt und in ihrer Raumausrichtung berechnet werden.

Ebenfalls ist eine Kombination aus den beiden oben genannten Möglichkeiten zur Kalibrierung der Raumrichtung der Schneidenkante möglich. Durch die entstehende Redundanz ist ein Plausibilitätscheck möglich. So kann zum Beispiel die Rotationsachse berechnet und mit der durch die Nutanlage ermittelten Position abgeglichen werden. Bei in etwa richtiger Positionierung (eingehaltener Schwellwert) werden die Geometriedaten des Kalibrierungsmittels für die Kalibrierung verwendet.

Als nächstes wird aufgezeigt, wie beispielsweise Schneidebenen kalibriert werden können, wenn der Meißel Schrägflächen aufweist, die im Weiteren auch als Schneidebenen bezeichnet werden. Hierbei ist festzustellen, wie die Schneidebenen liegen und unter welchem Winkel sie sich schneiden.

Eine Möglichkeit besteht darin, die Schneidebenen an Ebenen des Kalibrierungsinstrumentes anzulegen, wie sie in Figur 5 bezeichnet sind. Der Meißel wird mit seinen Schneidflächen in zwei aufeinanderfolgenden Kalibrierungsschritten an eine der Ebenen, zum Beispiel Ebene A, angelegt. Durch die bekannten Geometriedaten des Kalibrierungsinstrumentes bzw. der Ebene A können die Schneidebenen kalibriert werden. Wenn die Schneidebenen in sehr spitzem Winkel zueinander stehen (unendlich dünner Meißel) genügt unter Umständen die Kalibrierung einer Schneidebene.

Es ist, wenn ein Meißel mit einer Referenzeinrichtung im Navigationssystem verwendet wird, auch möglich, beide Schneidebenen in einem einzigen Schritt zu kalibrieren. Dies gilt dann, wenn die von der Referenz aufgespannte Ebene parallel und mit bekanntem Abstand zum Meißel-Achsenvektor 19 (Figur 4) liegt. Es genügt dann, die Ebene der Referenzanordnung bis zur Mitte des Meißelgriffs hin zu verschieben und die Schnittgerade mit der Ebene des Kalibrierungsinstruments (zum Beispiel Ebene A) zu berechnen. Durch eine Spiegelung an der Mittelebene (verschobene Referenzebene) ist dann auch die andere Schneidebene kalibriert.

Mit der erfindungsgemäßen Vorrichtung bzw. dem erfindungsgemäßen Verfahren lassen sich aber auch noch weitere Eigenschaften des Meißels kalibrieren, beispielsweise die Breite seiner Schneidenkante 2. Wenn wie oben beschrieben die Schneidebenen eines Meißels durch zweimaliges Anlegen an die Ebene A in ihrem Winkel zueinander bestimmt werden können, so besteht hierbei die Möglichkeit, auch die Breite der Schneidenfläche 2 gleich mitzubestimmen, und zwar dann, wenn - wie in Figur 5 dargestellt ist - die Schneidenkante 2 gleichzeitig im Anschlag an die Ebene C angelegt wird. Ein zweimaliges Anlegen des Meißels von jeder Flachseite her gestattet dann die Berechnung der Meißelbreite bzw. der Lage des Achsenvektors 19 des Griffstücks 23.

Eine Kalibrierung des Meißelgriffstücks 23 ist aber auch unter Verwendung der V-Nut 14 möglich, die am Instrumententeil 20 in Figur 3 gezeigt ist:

Die Kalibrierung des Achsenvektors 19 und des Radius des Meißelgriffabschnitts erfolgt durch ein Einsetzen in die V-Nut 14 und ein Ausführen einer kontinuierlichen Drehbewegung um die Rotationsachse des Meißels. Eine fest am Instrument angebrachte Referenz (Reflektorenanordnung) liefert dabei dem Trackingsystem kontinuierlich Daten (Samples). Diese werden durch computerunterstützte Graphikverarbeitung auf einen Zylinder gematcht. Der Zylinder hat zunächst einen beliebigen Radius. Seine Achse liegt jedoch parallel zur V-Nut 14 und auf der Ebene, die durch die V-Nut 14 und einen darauf senkrecht stehenden, den V-Nut-Öffnungswinkel halbierenden Vektor aufgespannt wird. Der zunächst variable Radius des Zylinders ist nach erfolgten optimiertem Match (zum Beispiel kleinster quadratischer Fehler, Standardabweichung als Plausibilitätscheck) der kalibrierter Radius des rotationssymmetrischen Teils. Für den Fall einer ungenauen Kalibrierung kann mit Hilfe eines manuell eingegebenen Radius bei nochmals erfolgendem Zylindermatch die Genauigkeit der Kalibrierung des Vektors erhöht werden.

Ist der Meißel-Griffabschnitt 23 dann kalibriert, kann mittels der oben schon beschriebenen Technik, d. h. mit Hilfe eines seitlichen Anschlages (Ebene C in Figur 5) die Meißelbreite kalibriert werden, wobei nur eine Seite der Schneidenkante an der Ebene C anliegen muss, da der Achsenvektor 19 bekannt ist. Mit dem obigen Schritt sind nunmehr alle notwendigen Kalibrierungen am Instrument vorgenommen.

Im Weiteren erfolgt die Beschreibung einer Kalibrierung eines Meißels (Schneidenkanten-Richtung im Raum und Griffabschnitt-Kalibrierung), die in lediglich einem einzigen Kalibrierungsschritt stattfinden kann.

Wenn nämlich sichergestellt ist, dass eine durch eine Referenz (Reflektorenadapter) aufgespannte Ebene parallel zum Vektor 8, 18 der Schneidenkante 2 liegt, kann auf die Einzelkalibrierung der Schneidenkanten-Ausrichtung im Raum verzichtet werden. Dann muss, wie oben zuletzt beschrieben, der Achsenvektor 19 des Griffabschnittes 23 in Verbindung mit seiner Länge kalibriert werden, was beispielsweise durch die Verwendung des Anschlags 17 möglich wird, der in Figur 3 gezeigt ist. Die Lage und Orientierung des Kantenvektors 18 im Raum errechnet sich dann durch die vorgegebene Parallelität zu der von der Referenz aufgespannten Ebene 6 und aus dem Achsenvektor 19 des Instruments, der dem Kantenvektor 18 unter senkrechtem Winkel schneidet. Die Länge der Schneidenkante wird dann entweder manuell eingegeben oder durch Anlegen an die Begrenzungsebene C (Figur 5) wie oben beschrieben ermittelt.

## Patentansprüche

1. Verfahren zur Kalibrierung bzw. Registrierung medizinischer Instrumente (1, 4) , bei dem eine räumliche Position des Instruments (1, 4) mittels eines medizinischen Navigationssystems ermittelt wird, um die Relativposition des Instruments (1, 4) bzw. gegenüber anatomischen Daten zu ermitteln, **dadurch gekennzeichnet, dass** die räumliche Ausrichtung eines linienförmigen oder ebenenförmigen, funktionellen Abschnitts (2) des Instruments (1, 4) ermittelt wird, wobei ein geeigneter Abschnitt des Instruments (1) an einem Kalibrierungsabschnitt (3', 9) eines Kalib- rierungshilfsmittels (3, 12) ausgerichtet wird, dessen räumliche Ausrichtung im Navigationssystem bekannt ist.

2. Verfahren nach Anspruch 2, bei dem die räumliche Ausrichtung der Schneidenfläche (2) eines chirurgischen Meißels (1, 4) ermittelt wird.

3. Verfahren nach Anspruch 1, bei dem der funktionelle Abschnitt (2) an dem Kalibrierungshilfsmittel (3, 9) bzw. an dessen Kalibrierungsabschnitt (3', 9) ausgerichtet wird.

4. Verfahren nach Anspruch 3, bei dem der Kalibrierungsabschnitt eine Linienform, insbesondere die Form einer Liniennut aufweist.

5. Verfahren nach Anspruch 3, bei dem der Kalibrierungsabschnitt eine Ebene ist.

6. Verfahren nach einem der Ansprüche 1 oder 2, bei dem die räumliche Ausrichtung des funktionellen Abschnitts (2) des Instruments (4) mit Hilfe schon vorhandener Informationen über die Ausrichtung einer bestimmten anderen charakteristischen Linie oder Ebene (6) für das Instrument (4) ermittelt wird.

7. Verfahren nach einem der Ansprüche 1 oder 2, bei dem die räumliche Ausrichtung des mehrdimensional ausgestalteten, funktionellen Abschnitts (2) des Instruments (1) mit Hilfe der Registrierung mehrerer Punkte auf dem Instrument (1) durch ein schon kalibriertes Registrierungsinstrument (11) ermittelt wird.

8. Vorrichtung zur Kalibrierung bzw. Registrierung medizinischer Instrumente (1, 4) mit einem medizinischen Navigationssystem, mit einem Kalibrierungshilfsmittel (3, 12), **dadurch gekennzeichnet, dass** das Kalibrierungshilfsmittel einen Kalibrierungsabschnitt (3', 9) aufweist, dessen räumliche Ausrichtung im Navigationssystem bekannt ist und an dem ein linien- oder ebenenförmigen funktioneller Abschnitts (2) des Instruments (1, 4) ausgerichtet werden kann.

9. Vorrichtung nach Anspruch 8, **dadurch gekennzeichnet, dass** der Kalibrierungsabschnitt die Form einer Liniennut aufweist.

10. Vorrichtung nach Anspruch 8, **dadurch gekennzeichnet, dass** der Kalibrierungsabschnitt eine Ebene ist.

11. Vorrichtung nach Anspruch 8, **dadurch gekennzeichnet, dass** der Kalibrierungsabschnitt mehrere Begrenzungsebenen und die von diesen eingeschlossenen Schnittlinien oder Schnittpunkte aufweist.

## Claims

1. A method for calibrating and/or registering medical instruments (1, 4), in which a spatial position of the instrument (1, 4) is determined by means of a medical navigation system, in order to determine the relative position of the instrument (1, 4) with respect to anatomical data, **characterised in that** the spatial orientation of a linear or planar functional section (2) of the instrument (1, 4) is determined, wherein a suitable section of the instrument (1) is aligned on a calibration section (3', 9) of a calibration aid (3, 12) whose spatial orientation in the navigation system is known.

2. The method according to claim 1, wherein the spatial orientation of the cutting area (2) of a surgical chisel (1, 4) is determined.

3. The method according to claim 1, wherein the functional section (2) is aligned on the calibration aid (3, 12) and/or on its calibration section (3', 9).

4. The method according to claim 3, wherein the calibration section exhibits a linear form, in particular the form of a linear groove.

5. The method according to claim 3, wherein the calibration section is a plane.

6. The method according to any one of claims 1 or 2, wherein the spatial orientation of the functional section (2) of the instrument (4) is determined with the aid of information already available on the orientation of another particular characteristic line or plane (6) of the instrument (4).

7. The method according to any one of claims 1 or 2, wherein the spatial orientation of the multi-dimensionally formed, functional section (2) of the instrument (1) is determined with the aid of registering a number of points on the instrument (1) using an already calibrated registering instrument (11).

8. A device for calibrating and/or registering medical instruments (1, 4) using a medical navigation system, comprising a calibration aid (3, 12), **characterised in that** the calibration aid comprises a calibration section (3', 9) whose spatial orientation in the navigation system is known and on which a linear or planar functional section (2) of the instrument (1) can be orientated.

9. The device according to claim 8, **characterised in that** the calibration section exhibits the form of a linear groove.

10. The device according to claim 8, **characterised in that** the calibration section is a plane.

11. The device according to claim 8, **characterised in that** the calibration section comprises a number of boundary planes and the intersecting lines or intersecting points enclosed by them.

## Revendications

1. Procédé pour étalonner ou enregistrer des instruments médicaux (1, 4), dans lequel une position spatiale de l'instrument (1, 4) est déterminée au moyen d'un système de navigation médical afin de déterminer la position relative de l'instrument (1, 4) ou des données anatomiques concernant celui-ci, **caractérisé par** l'étape consistant à déterminer l'orientation spatiale d'une partie fonctionnelle en forme de ligne ou en forme de plan (2) de l'instrument (1, 4), une partie adaptée de l'instrument (1) étant orientée sur une partie d'étalonnage (3', 9) d'une aide à l'étalonnage (3, 12) dont l'orientation spatiale dans le système de navigation est connue.

2. Procédé selon la revendication 2, comportant l'étape consistant à déterminer l'orientation spatiale des surfaces d'intersection (2) de ciseaux chirurgicaux (1, 4).

3. Procédé selon la revendication 1, comportant l'étape consistant à orienter la partie fonctionnelle (2) sur l'aide à l'étalonnage (3, 9) ou sur sa partie d'étalonnage (3', 9).

4. Procédé selon la revendication 3, dans lequel la partie d'étalonnage a une forme de ligne, en particulier la forme d'une gorge linéaire.

5. Procédé selon la revendication 3, dans lequel la partie d'étalonnage est un plan.

6. Procédé selon l'une quelconque des revendications 1 ou 2, comportant l'étape consistant à déterminer l'orientation spatiale de la partie fonctionnelle (2) de l'instrument (4) à l'aide d'informations déjà existantes concernant l'orientation d'une autre ligne ou d'un autre plan caractéristique déterminé (6) pour l'instrument (4).

7. Procédé selon l'une quelconque des revendications 1 ou 2, comportant l'étape consistant à déterminer l'orientation spatiale de la partie fonctionnelle configurée de manière multidimensionnelle (2) de l'instrument (1) par enregistrement de plusieurs points sur l'instrument (1) au moyen d'un instrument d'enregistrement déjà étalonné (11).

8. Dispositif pour étalonner ou enregistrer des instruments médicaux (1, 4), muni d'un système de navigation médical, d'une aide à l'étalonnage (3, 12), **caractérisé en ce que** l'aide à l'étalonnage comporte une partie d'étalonnage(3', 9) dont l'orientation spatiale dans le système de navigation est connue et sur laquelle une partie fonctionnelle en forme de ligne ou de plan (2) de l'instrument (1, 4) peut être orientée.

9. Dispositif selon la revendication 8, **caractérisé en ce que** la partie d'étalonnage a la forme d'une gorge linéaire.

10. Dispositif selon la revendication 8, **caractérisé en ce que** la partie d'étalonnage est un plan.

11. Dispositif selon la revendication 8, **caractérisé en ce que** la partie d'étalonnage comporte plusieurs plans de délimitation et les lignes d'intersection ou points d'intersection entourés par ceux-ci.
